(19) Europäisches Patentamt
European Patent Office
Office européen des brevets

(11) **EP 4 265 289 A1**

(12) **EUROPEAN PATENT APPLICATION**

(43) Date of publication:
**25.10.2023  Bulletin 2023/43**

(21) Application number: **22171395.1**

(22) Date of filing: **03.05.2022**

(51) International Patent Classification (IPC):
**A61M 16/00** (2006.01)   **A61M 16/06** (2006.01)
**A61M 16/12** (2006.01)   **A61M 16/10** (2006.01)

(52) Cooperative Patent Classification (CPC):
(C-Sets available)
**A61M 16/0003; A61M 16/024; A61M 16/0666;**
**A61M 16/1005; A61M 16/125;** A61M 16/0066;
A61M 2016/0027; A61M 2202/0225;
A61M 2205/3334; A61M 2205/70; A61M 2230/202;
A61M 2230/205; A61M 2230/42; A61M 2230/432

(Cont.)

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority:  **21.04.2022   US 202263333269 P**

(71) Applicant: **Koninklijke Philips N.V.**
**5656 AG Eindhoven (NL)**

(72) Inventors:
• **BOU JAWDE, Samer**
  **Eindhoven (NL)**
• **DE GRAAF, Pascal**
  **Eindhoven (NL)**
• **BRANS, Harold Johannes Antonius**
  **Eindhoven (NL)**

(74) Representative: **Philips Intellectual Property &**
**Standards**
**High Tech Campus 52**
**5656 AG Eindhoven (NL)**

(54) **HIGH FLOW NASAL THERAPY SYSTEM AND METHOD**

(57)     A therapy system has a nasal cannula patient interface configured to deliver gas to a nasal cavity of a patient and a delivery system for delivering oxygen-enriched breathing gas, comprising air and enrichment oxygen, to the patient interface. A sensor is provided allowing arterial partial pressure of CO2, PaCO2 to be measured or estimated thereby to generate capnograph data, A parameter is determined from the capnograph data which is representative of CO2 washout, and the total flow rate, the oxygen flow rate or the air flow rate of the breathing gas delivered by the delivery system are iteratively adjusted while monitoring the determined parameter. A value of total flow rate, oxygen flow rate or air flow rate is used which maximizes CO2 washout or achieves a desired level of CO2 washout.

FIG. 2

(52) Cooperative Patent Classification (CPC): (Cont.)

C-Sets
A61M 2202/0225, A61M 2202/0085;
A61M 2230/202, A61M 2230/005;
A61M 2230/42, A61M 2230/005

**Description**

FIELD OF THE INVENTION

The invention relates to High Flow Nasal Therapy (HFNT).

BACKGROUND OF THE INVENTION

HFNT provides high flows to patients through a nasal cannula. The flow rate is typically higher than as provided by conventional oxygen support. The basic idea is an extension to traditional oxygen support. Oxygen therapy typically provides only oxygen at low rates that usually range from 1 to 15 L/min. The oxygen is delivered via a so-called patient interface, which may comprise a nasal cannula, a face mask, a venturi mask, or a reservoir bag mask.
HFNT has two high pressure sources, for an air flow and for an oxygen flow. In combination, these high flows can reach a total of 60 L/min.
HFNT therapy is typically delivered through a device that has two main controls, the first being a control of the total rate of gas flow and the second being a control over the percentage of oxygen delivered compared to the percentage of air. Thus by varying one or both controls, one or more of the total flow rate, oxygen flow rate or air flow rate may be adjusted. The oxygen support is provided because HFNT was initially introduced as an elevated therapy for adults already on oxygen therapy (at normal flow rates) and for patients who are most likely hypoxemic or suffer from hypoxemic respiratory failure.
The basic device includes an air/oxygen blender and a heated humidifier to make sure the air is delivered at the appropriate temperature and humidity to the patient, mainly for comfort. With the blender, the fraction of inspired oxygen (FiO2) is for example controllable to range from 21% to 100%.
The HFNT device is reported as offering several potential benefits, including:

maintenance of a constant Fi02;
generation of a positive end-expiratory pressure (PEEP) and increased end-expiratory lung volume;
reduction of the anatomical dead space;
improvement of mucociliary clearance;
facilitation of carbon dioxide (CO2) washout from the airway system; and
reduction in the breathing frequency and work of breathing.

Additionally, only requiring a nasal cannula instead of a mask means that HFNT provides much more comfort to the patients and allows them to perform more naturally their daily activities (e.g. eating, drinking, etc.).
Unfortunately, the primary use of HFNT is limited to stable hypoxemic patients (i.e. those with very low oxygen levels in their blood), which restricts it to a limited patient population and also mainly restricts it to in-hospital usage.
HFNT is also referred to using several additional terms. The terms include high flow nasal cannula (HFNC) and nasal high flow (NHF). Herein the terminology HFNT shall be used throughout but shall be understood as equivalent to HFNC or HNF.
Patent application US2011/0257550 discloses a method and apparatus for continuous monitoring of exhaled carbon dioxide in a gas supply system. The apparatus has a general mask interface to measure exhaled carbon dioxide (CO2) via a nasal cannula/interface.

SUMMARY OF THE INVENTION

The invention is defined by the claims.
According to examples in accordance with an aspect of the invention, there is provided a therapy system, comprising:

a patient interface adapted to deliver gas to a nasal cavity of a patient;
a delivery system for delivering an oxygen-enriched breathing gas to the patient interface, wherein the delivery system is adapted to deliver a total flow rate of breathing gas comprising an oxygen flow rate and an air flow rate, wherein the delivery system is controllable to adjust one or more of the total flow rate, the oxygen flow rate and the air flow rate;
a controller for controlling the delivery system;
a sensor arrangement adapted to enable measurement or estimation of the arterial partial pressure of arterial carbon dioxide, PaCO2, of the patient over time and thereby generate capnograph data; and
wherein the controller is adapted to:

iteratively:
determine a parameter from the capnograph data, which parameter is representative of CO2 washout;
adjust one or more of the total flow rate, the oxygen flow rate or the air flow rate of the breathing gas delivered by the delivery system; and
determine the impact of the adjusting upon the determined parameter, in order to determine a value of total flow rate, oxygen flow rate or air flow rate which maximizes CO2 washout or achieves a desired level of CO2 washout; and
set the total flow rate, oxygen flow rate or air flow rate at the determined value.

The therapy system enables the functionality of a standard high flow nasal therapy (HFNT) system to be extended by measuring or estimating the arterial CO2 partial pressure, thereby generating capnograph data, and by using features of the capnograph data to adjust the oxygen flow, the air flow or the total flow. This enables the system to achieve maximum or target CO2 washout by adjusting the operating parameters of the delivery system. As a result, the therapy system becomes a more reliable treatment option both in the home and in a clinical setting. The system may permit delivery of better therapy to current HFNT-treated patients as well as expand the use of HFNT devices to other patient conditions. For example, the system becomes suitable for home treatment of hypercapnic COPD or OSA patients.

The capnograph data is a set of data indicating partial pressure of CO2 over time or with respect to volume (however, the volume varies over time so these representations are essentially equivalent), in particular during inspiration and expiration. It does not require the actual generation of a graphic output, but is the data that would enable a capnograph to be generated.

Conventional HFNT therapy facilitates removal of CO2 from the airway system, the removal of which is beneficial for effective ventilation. This removal is commonly referred to as CO2 washout. In conventional therapy, CO2 washout is achieved to a certain point, but CO2 washout it not maximized. This is because air and oxygen flows are manually set accordingly to typical therapy values, and the conventional HFNT device provides no means to determine or optimize CO2 washout. Through the use of capnogram data, the present invention permits air and oxygen flows to be adjusted in order to achieve a maximum or target CO2 washout. The current disadvantages of HFNT are that (1) it is restricted to stable hypoxemic patients and (2) it does not highly depend on measured CO2 concentrations (e.g. exhaled or transcutaneous) to diagnose and optimize the therapy of these patients. Through this invention, control of HFNT settings is further refined not only to provide better therapy for hypoxemic patients but also to provide the benefits of comfort and simplicity of HFNT to other patient populations such as COPD and OSA who suffer from hypercapnia (and thus would benefit from CO2 monitoring) and have much higher prevalence and dominance in the population (in comparison to stable hypoxemic patients).

In capnography, capnographs are generated by plotting the pressure of CO2 from exhaled gases in two main ways: either as a time capnography plot where the partial pressure of expired respiratory CO2 (PrCO2) is plotted as a function of time, or as a volume capnography plot where PrCO2 is plotted as function of exhaled volume. In either case the last value of PrCO2 is the end-tidal CO2 concentration (EtCO2) and may be used to estimate PaCO2.

Since capnography provides information on expiratory-gas levels (including breathing phase), the capnograph data can provide a lot of information on respiratory mechanics (such as disease progression or phase). Capnography may be used for inhalation exhalation (IE) detection or to estimate respiratory mechanics, enabling improved HFNT therapy by tailoring the therapy to patient needs.

With microstream (trademark) technology, a side-stream (i.e. diverted flow) capnograph can be generated from the nasal cannula and requires an exhalation flow rate which may be as low as 50 ml/min to produce an estimate of EtCO2. Microstream technology is discussed for example in Colman, Yehoshua, and Baruch Krauss. "Microstream Capnography Technology: A New Approach to an Old Problem," n.d., 7.

The arterial partial pressure can for example be measured using exhaled CO2 measurements via capnography. Based on one or more features of the capnograph data, the oxygen and air (or the total flow, which is a combination of both) may be controlled to obtain either maximum CO2 washout, or a configurable CO2 washout which may be set by an operator via a user interface.

The process of determining optimum oxygen or air flow settings is an iterative process whereby capnograph data is generated and one or more features are captured, one or more of the oxygen and air flow settings are changed, subsequent capnograph data is generated and the impact upon the one or more features of the subsequent capnograph data is determined, and settings are continually adjusted until desired air or oxygen flow settings are achieved, that is, settings which optimize a feature of the capnogram data and therefore maximize or control CO2 washout.

For example, a first iteration series may determine an optimum air flow, which may then be set, and a second iteration series may then determine an optimum oxygen flow, which may also then be set. Alternatively a first iteration series may determine an optimum oxygen flow, which may then be set, and a second iteration series may then determine an optimum air flow, which may also then be set.

Two possible approaches to maximizing CO2 washout, according to features of the capnogram data, are to maximize the phase two angle (which indicates optimum PEEP), or to maximize end-tidal $CO_2$ concentration. The phase two angle is known in the art, and is the angle between the slopes of phases II and III. The slope of a capnograph (i.e. the rate of $PaCO_2$ against time/volume when considering the underlying capnograph data) changes significantly between phases II and III. The end-tidal $CO_2$ concentration, $EtCO_2$, is known in the art and is the value of $PaCO_2$ ad the end of phase III (i.e. the maximum value of $PaCO_2$ when considering the underlying capnograph data).

It is not necessary to measure PEEP directly in order to achieve maximum $CO_2$ washout, but it may be useful to do so, for example to determine a numerical value of PEEP associated with maximum $CO_2$ washout. PEEP may be measured through the use of an appropriate pressure sensor.

Monitoring $CO_2$ and determining one or more capnogram features enables better therapy to be delivered to current HFNT-treated patients as well as expanding the use of HFNT devices to other patient conditions (e.g. hypercapnic). In additional to maximizing $CO_2$ washout this approach may help set the optimal PEEP, avoid hyperventilation, reduce deadspace, and enhance the use of HFNT to other patients such as COPD or OSA patients.

The measured parameter from the capnograph data may be the end-tidal $CO_2$ concentration, $EtCO_2$, and the controller is adapted to determine the value of total flow rate, oxygen flow rate or air flow rate for maximizing $CO_2$ washout by maximizing the value of $EtCO_2$.

$EtCO_2$ is a simple value to determine from the capnograph data and is a reliable measurement and provides a reliable means to maximize $CO_2$ washout. It may be additionally useful to determine a calibration factor when optimizing the $EtCO_2$.

The measured parameter of the capnograph data may be the phase two angle, and the controller may be adapted to determine the value of total flow rate, oxygen flow rate or air flow rate to optimize PEEP to provide the maximum phase two angle, in order to achieve a maximum $CO_2$ washout.

Phase two angle is a simple value to determine from the capnograph data and provides a reliable means to maximize $CO_2$ washout.

The controller may be further adapted to: derive, from the measurement or estimation of the arterial partial pressure, a patient parameter or index; monitor the patient parameter or index over time to detect or monitor a patient condition; and adjust one or more of the total flow rate, the oxygen flow rate or the air flow rate of the breathing gas delivered by the delivery system in dependence on the detected or monitored patient condition.

The monitoring a patient parameter or index over time may comprise monitoring one or more of end-tidal $CO_2$ concentration, $EtCO_2$, a rapid shallow breathing index, RSBI, or a ratio of $SpO_2$ / $FiO_2$ to respiratory rate, ROX index, or one or more parameters derived from the capnography waveform

Monitoring of such a patient parameter or index allows the oxygen flow, air flow or total flow of the therapy system to be optimally configured. For example: for a high rapid shallow breathing index (RSBI), the device could increase the total flow to provide more support; for a low $SPO_2$ / $FIO_2$ ratio it could provide higher $FIO_2$, and if there is no improvement eventually set an alarm or otherwise alert the therapist; $EtCO_2$ could be high because there is too much dead space (which may be due to the PEEP being too high), or PEEP may be too low and there may not be enough support, and thus the device could alter the PEEP level and monitor the impact upon the patient.

Thus, various different parameters may be monitored, and these may provide information relating to the type and severity of a range of different diseases and their progression. For example, if a capnograph is used, the slope of different phases is related to asthmatic patients and their severity. Different monitored parameters for example enable differentiation between COPD patients and congestive heart failure patients.

The sensor arrangement may be further adapted to measure or estimate $SpO_2$ over time, and the controller may be further adapted to receive or determine a target $SpO_2$ level and adjust one or more of the total flow rate, oxygen flow rate or air flow rate of the breathing gas in dependence on measured or estimated $SpO_2$ level to achieve the target $SpO_2$ level.

Additionally receiving or determining a target $SPO_2$ level and measuring or estimating $SpO_2$ can help achieve the target $SpO_2$ level and help determine an optimal PEEP level. Known HFNT systems, which control only $FiO_2$ and monitor only $SpO_2$ do not guarantee optimal PEEP levels.

The controller may be adapted to first adjust the air flow to achieve the optimal air flow rate for maximum $CO_2$ washout, then adjust the oxygen flow to achieve the target $SpO_2$ level.

This allows the maximum $CO_2$ washout to be achieved whilst also reaching or attempting to reach the target $SpO_2$ level. The target $SpO_2$ level may be a range, and may be set by an operator using an appropriate interface, or may be a maximum $SPO_2$ level, which may be preconfigured, or manually set or confirmed by an operator. In addition to maximizing $CO_2$ washout, it may be additionally desirable to achieve a particular $SpO_2$ level, which, depending on patient needs, may be either a maximum $SpO_2$ level or a target level set by the operator.

Measurements of exhalation partial pressure may be for example obtained from the capnograph data or measured directly. *While transcutaneous monitoring provides information on blood-gas level, the capnograph data can provide $\alpha$ lot of information on respiratory mechanics (such $\alpha$s disease progression or phase).*

The controller may be adapted to estimate the arterial partial pressure resulting from the measurement of the diluted exhalation partial pressure, by generating a calibration factor as a function of time.

The controller may be adapted to compensate for dilution of the flow by constructing adjusted capnograph data and estimating a calibration factor, wherein the estimating a calibration factor comprises generating a first set of capnograph data and a second set of capnograph data, and the controller may be adapted to control the sensor arrangement and the total flow rate of the delivery system to generate the first set of capnograph data and the second set of capnograph data at different flows.

When the delivered flow is not zero, the measured CO2 concentration from the capnograph data is not entirely accurate, as it is diluted by exhaled air mixing with excess flow of the HFNT device. It is beneficial to estimate or determine this dilution factor. It is possible to derive the dilution factor by also taking a measurement when the total flow is zero (i.e. when there is no dilution). Further, taking several measurements would provide better estimates for the dilution factor.

The controller may be adapted control the sensor arrangement and the total flow rate of the delivery system to generate either the first set of capnograph data or the second set of capnograph data when the total flow is zero.

Taking one of the measurements when the total flow is zero ensures that an accurate reading may be taken of exhaled CO2 concentration, thereby allowing effective calibration.

The sensor arrangement may be adapted to measure an intrinsic PEEP, iPEEP, level and wherein the controller is adapted to determine a target positive end-expiratory pressure, PEEP, taking into account the measured iPEEP level.

The invention also provides a computer-implemented method for controlling the delivery of gas to a nasal cavity of a patient via a patient interface, the method comprising:

> controlling a delivery system to deliver an oxygen-enriched breathing gas to the patient interface, thereby delivering a total flow rate of breathing gas comprising an oxygen flow rate and an air flow rate;
> iteratively:
>
>> receiving capnograph data from a sensor arrangement;
>> determining a parameter from the capnograph data, which parameter is representative of CO2 washout;
>> adjusting one or more of the total flow rate, the oxygen flow rate or the air flow rate of the breathing gas delivered by the delivery system; and
>> determining the impact of the adjusting upon the determined parameter, in order to determine a value of total flow rate, oxygen flow rate or air flow rate which maximizes CO2 washout or achieves a desired level of CO2 washout; and
>> setting the total flow rate, oxygen flow rate or air flow rate at the determined value.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

> Fig. 1 shows a prior art therapy system for high flow nasal therapy;
> Fig. 2 shows a therapy system for high flow nasal therapy according to the invention;
> Fig. 3 shows how a capnograph is used;
> Fig. 4 shows a time capnograph;
> Fig. 5 shows a volume capnograph;
> Fig. 6 shows how air and oxygen flows may be controlled;
> Fig. 7 shows how controlled parameters may be adjusted according to changes in targeted parameters;
> Fig. 8 shows a phase 2 angle (from a volume capnograph) plot against PEEP;
> Fig. 9 shows a plot of EtCO2 or ROX over time;
> Fig. 10 shows a plot of PEEP or multiple settings over time;
> Fig. 11 shows plots of PrCO2, patient effort and flows over time;
> Fig. 12 shows a capnograph with an indent;
> Fig. 13 shows a capnograph with the PrCO2 plot of two breaths superposed;
> Fig. 14 shows a capnograph with an extrapolated PrCO2 plot; and
> Fig. 15 shows a plot of dilution calibration factor against total flow.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a therapy system which has a nasal cannula patient interface configured to deliver gas to a nasal cavity of a patient and a delivery system for delivering oxygen-enriched breathing gas, comprising air and enrichment oxygen, to the patient interface. A sensor is provided allowing arterial partial pressure of CO2, PaCO2 to be measured or estimated thereby to generate capnograph data. A parameter is determined from the capnograph data which is representative of CO2 washout, and the total flow rate, the oxygen flow rate or the air flow rate of the breathing gas delivered by the delivery system are iteratively adjusted. A value of total flow rate, oxygen flow rate or air flow rate is used which maximizes CO2 washout or achieves a desired level of CO2 washout.

The terms capnograph waveform, capnograph, and waveform as used throughout this document are equivalent. Throughout this document, usage of O2 is equivalent to $O_2$ and usage of CO2 is equivalent to $CO_2$, etc. For example, PaCO2 means PaCO2, etc.

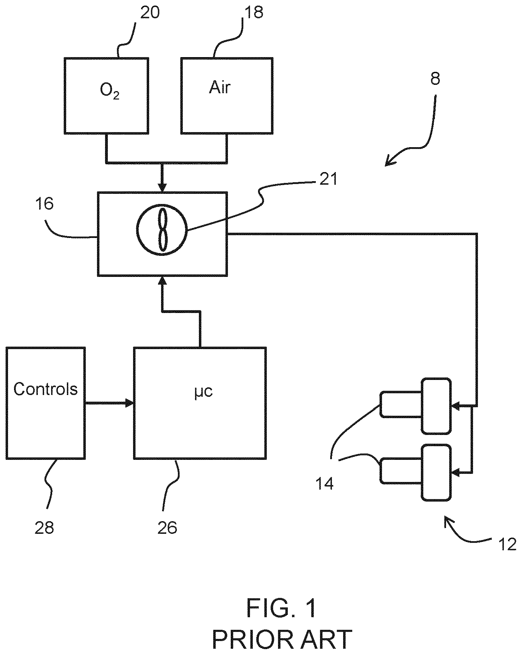

Fig. 1 shows therapy system for high flow nasal therapy as is known in the art in schematic form.

The therapy system 8 comprises a nasal cannula patient interface 12 configured to deliver gas to a nasal cavity of a patient. The patient interface may comprise a pair of nose prongs 14.

A delivery system 16 is provided for delivering oxygen-enriched breathing gas, comprising ambient or compressed air 18 and enrichment oxygen 20, to the patient interface 122. The delivery system for example comprises a flow pump (blower 21). Control signals 28 are processed by a microcontroller 26, which controls a valve arrangement (not shown) to vary the coupling of ambient air and/or enrichment oxygen to the patient interface. The delivery system 16 and associated microcontroller control 26 thus function as an air / oxygen blender. The control signals 28 are manually configured to provide an appropriate air flow, oxygen flow and total flow from the therapy system. The control signals 28 may be manually adjusted periodically (every few weeks or months), if required, according to a medical professional's ongoing review of the patient's condition and their respiratory needs.

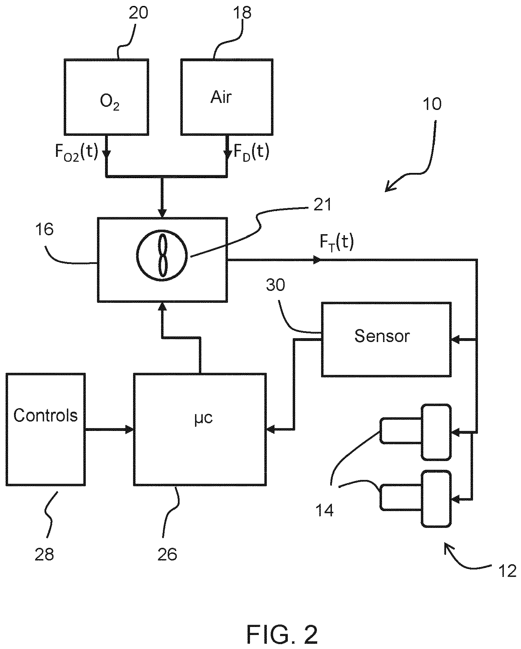

Fig. 2 shows therapy system for high flow nasal therapy according to the invention in schematic form. Like features are equivalent to those in Fig. 1. Oxygen flow, $F_{O2}(t)$, air flow, $F_D(t)$, and total flow, $F_T(t)$,, each a function of time, are shown.

The therapy system 10 comprises a sensor arrangement 30 adapted to enable measurement or estimation of the arterial partial pressure of arterial carbon dioxide, PaCO2, of the patient overtime. In this way, capnograph data (as defined above) is generated. This may for example be a sensor for measuring PrCO2 for detecting CO2 in exhaled breath. Alternatively, the sensor arrangement may comprise a sensor configured to measure CO2 from a patient ear or from a patient finger. This may be a simple and easy way to measure CO2. The sensor data is stored in a memory (not shown) so that analysis of the data characteristics over time is possible, to determine parameters from the capnograph data, and in particular parameters that depend on, and hence are representative of, the level of CO2 washout.

This signal from this detector is passed to the microcontroller 26 which uses this information to determine the optimal oxygen flow, air flow and total flow to provide to the patient.

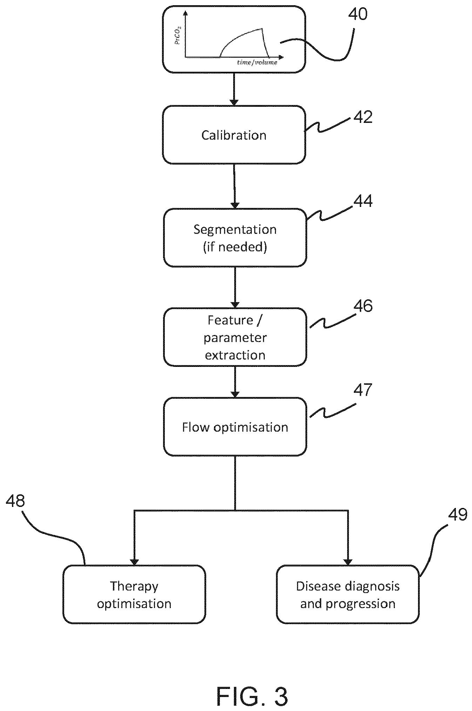

Fig. 3 shows how a capnograph 40 (or, in practice, the underlying capnograph data) is employed to assist therapy optimization or disease diagnosis.

The capnograph 40 is a measure of CO2 against time or volume, and may provide many features which may be useful for determining appropriate variations to HFNT settings, either for short term (i.e. immediate) therapy optimization or longer term disease diagnosis and HFNT control as discussed above. Data from a full capnograph 40 or partial capnograph may be used for calibration. The capnograph 40 may also be divided into its partial segments (i.e. phases) and features may be extracted. This information may be used, optionally along with other measurements, to optimize and monitor patient deterioration/improvement and therapy.

In step 42, an optional calibration step is carried out. This involves compensating for flow dilution caused by the flow from the HFNT device.

The capnograph is known to provide considerable insight on lung condition. For this purpose, the capnograph is segmented in step 44 and feature or parameter extraction takes place in step 46. In this way, following calibration due to flushing/dilution, segmentation of the capnograph takes place into its different phases and capnograph features are extracted from the curve (e.g. end-tidal CO2, EtCO2).

In step 47, the extracted features and parameters are used to tune flow settings. This is done by observing at least one feature or parameter, adjusting one or more of the total flow, air flow or oxygen flow, obtaining further capnograph data and determining the impact of the adjustment on the feature or parameter, and continuing to adjust flow until the feature or parameter has a value corresponding to a desired or maximum CO2 washout.

For example, the end-tidal CO2 (EtCO2) value may be taken from first capnograph data and the flow settings adjusted. Second capnograph data may be taken and the EtCO2 value again determined, and the impact upon the EtCO2 value determined. This iterative process may be repeated as many times as required to determine the flow settings which achieve a desired EtCO2 value, such as a maximum value, in order to achieve a desired level of CO2 washout or maximize CO2 washout.

As another example, the phase two angle may be taken from first capnograph data, one or more of the flow settings adjusted, and second capnograph data may be taken and the phase two angle again determined, and the impact of the adjustment on the upon the phase two angle determined. This iterative process may be repeated as many times as required to determine the flow settings which achieve a PEEP level at which the phase two angle is maximized, in order to maximize CO2 washout.

The flow optimization based on extracted features and parameters is used to provide therapy optimization in step 48 (optimizing HFNT settings) and to track disease diagnosis and progression (and adjusting HFNT settings accordingly) in step 49.

The therapy optimization may be an immediate response based on the features extracted and thus the controller 26 will act accordingly (e.g. change settings instantaneously). On the other hand, the controller 26 may not immediately change settings based on the disease diagnosis and progression (or monitoring). This data is utilized to alarm the patient and/or clinician of any adverse events, deterioration, or improvement following any changes in therapy (including HFNT settings and medication). Therefore, disease diagnosis and progression data may be stored upon a suitable medium. This could be stored in the device and ultimately uploaded to the cloud. The detail of such storage will be known to the skilled person and is not discussed in detail. Thus, according to the invention, HFNT settings may be immediately altered to optimize therapy, or may be adjusted over a much longer time period. Control of HFNT settings means one or more of the air flow, oxygen flow or total flow of the breathing gas, as required.

In its most basic form, HFNT allows the clinician to control the oxygen flow (to achieve a set Fi02) and the total flow. These two are often selected without the knowledge of their impact on parameters such as PaCO2 and nasal pressure as well as dead space and CO2 washout.

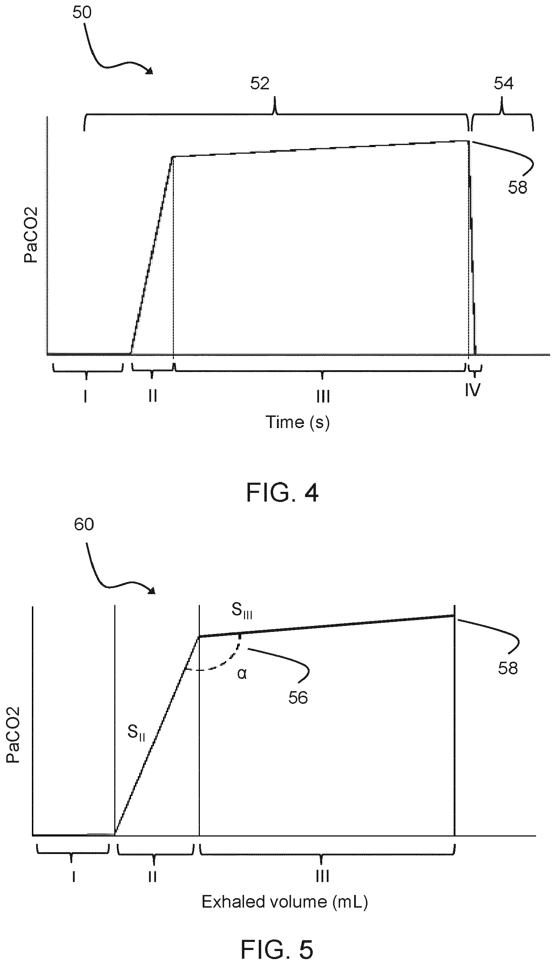

Fig. 4 shows a time capnograph 50. The partial pressure of CO2, PrCO2 is plotted against time. PrCO2 is plotted during an expiration period 52 and inspiration period 54. Phases I, II, III and IV are shown. Each of these different phases I, II, III, and IV provide insight on respiratory mechanics and blood-gas exchange. End-tidal CO2 (EtCO2) 58 is the level of CO2 at the end of the expiration period 52.

Fig. 5 shows a volume capnograph 60. The partial pressure of CO2 ,PrCO2 is plotted against exhaled volume. Phases I, II, and III are shown. Each of these different phases I, II, and III provide insight on respiratory mechanics and blood-gas exchange. End-tidal CO2 (EtCO2) 58 is the level of CO2 at the completion of exhalation. $S_{II}$ and $S_{III}$ are the slopes in phase II and III respectively. $\alpha$ is known as the phase II / phase two / phase 2 angle 56 and is the angle between the slopes of the plot during phases II and III.

Phase I, the only inspiratory phase, is usually a plateau in case of no rebreathing. It represents both the total inspiration cycle, almost free of CO2, and early expiration where the air exhaled is from the dead space where there was no mixing between fresh and alveolar air. In phase II, mixing is occurring between alveolar gas from both ventilated as well as shunted (alveolar dead space) and anatomical dead space. Thus PrCO2 is increasing. Furthermore, because of turbulent mixing and diffusion at the boundary of the dead space and alveolar gas, phase II includes gas that is part of phase I and III. Because of this physiology, the rise in slope during phase II captures the matching between perfusion and ventilation. The more abrupt it is, the better is the matching.

Many lung diseases, whether related to gas diffusion or pulmonary-vascular diseases, are associated with slower rise of this region or smaller slope. The gas from phase III is mainly contributed from the alveoli. Theoretically, at steady state in a healthy subject with identical alveoli and matching ventilation-perfusion ratio, at this phase, the PrCO2 can be estimated as the mean of the alveolar PCO2 (PACO2), thus the plot of PrCO2 - in particular the EtCO2 value - can be used to determine an estimate of arterial PCO2 (PaCO2).

Noting that breathing is phasic and PACO2 approaches venous and not arterial PCO2 (i.e. PaCO2) values, end-tidal PrCO2 (EtCO2) can properly estimate PaCO2 in healthy subjects (with sufficient expiratory time) but can be highly underestimated when ventilation is high, in the presence of alveolar dead space, and short expiratory times. The reason phase III is not an actual plateau but keeps slightly increasing is due to many reasons such as increasing PACO2, ventilation perfusion matching, rate of CO2 production, and positive end-expiratory pressure value.

The last phase, phase IV, is a sudden drop in PrCO2 due to the onset of inspiration.

The volume capnography plots the expiration volume only, and therefore it lacks an initial inspiratory phase and is composed of three phases. In phase I, the anatomic dead space empties. Thus, there is no change in PrCO2. Mixing between dead space gas and alveoli occurs in phase II, and then finally phase III captures the emptying of the alveoli. Thus the volume capnography provides physiological insight on dead space and alveolar tidal volume with better estimation of the perfusion/ventilation mismatch captured in the slope SIII of the phase III since the expired volume is an exponential decay. This larger slope may be an advantage for detection.

The slopes of the different capnograph phases may be related to asthmatic patients and their severity (divided into three groups). The capnograph can also be used to differentiate between normal, COPD, and Congestive Heart Failure (CHF). Furthermore, and in relationship with COPD, the different phases (and thus the progression of disease) could be tracked via volume capnography by calculating an efficiency index.

Features/parameters which could be extracted from the capnograph and utilized to optimize therapy, diagnose and assess the progression of a disease include but are not limited to:

Exhalation duration (duration of phases II + III);
Maximum partial pressure of carbon dioxide in exhaled breath (end-tidal concentration EtCO2);
Plateau time (duration of phase III);
End exhalation slope (SIII, i.e. the slope of phase III);
Respiratory rate (determined by the rate at which capnographs are generated);
Normalized time spent at plateau (plateau time divided by exhalation duration);
Slopes and slope ratios measured along the curve phases (slope of phase I, II, III, and ratios between theses slopes;
Second order derivatives measured along the curve phases (rate of change of slopes of I, II, III);
Area ratios measured across the curve (integration of the area under the curve for one or more of phases I, II, III);
Angle between ascending phase and plateau (phase II/2 angle, $\alpha$, 56, as measured between the slopes of phases II and III);

Quotient between exhaled $CO_2$ volume and the hypothetical $CO_2$ volume from a homogeneous lung (ratio between total exhaled CO2 and a reference number).

Where the term "capnograph" is used without specifying a type, it may be either a time capnograph 50 or a volume capnograph 60 according to circumstances.

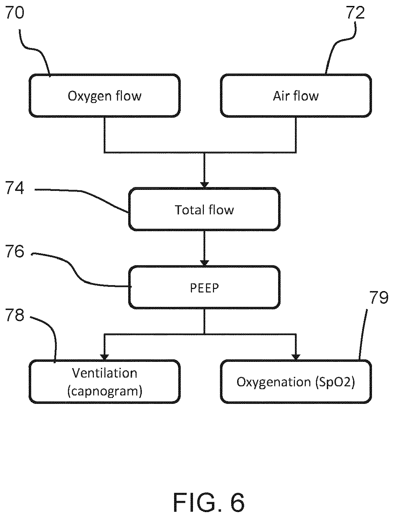

Fig. 6 shows how air and oxygen flows may be controlled, providing an illustrative example of how HFNT therapy could be optimized. In its current form, HFNT lacks a critical measurement to expand therapy beyond hypoxemic patients and properly offer an optimal personal treatment.

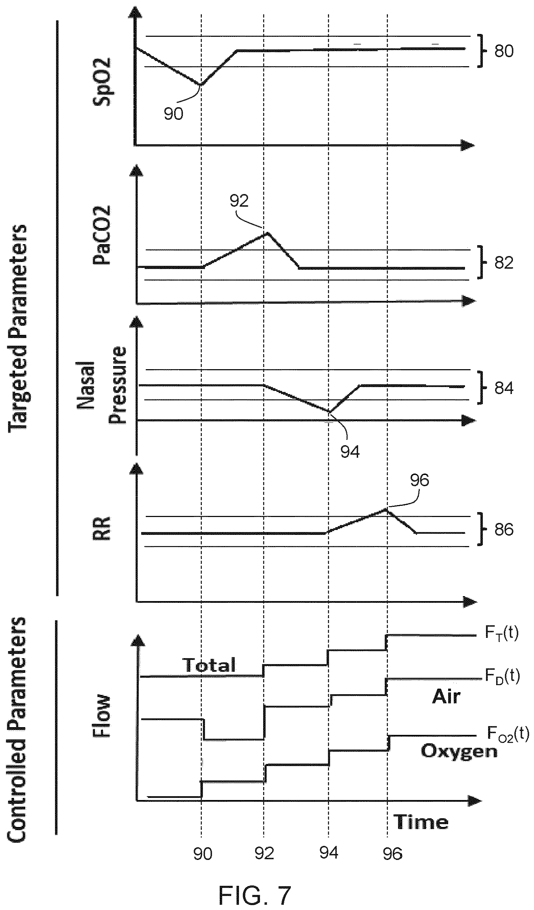

Currently HFNT controls oxygen flow, shown as step 70 in Fig. 7, and controls air flow shown as step 72. The total flow 74 can lead to PEEP which positively effects oxygenation as shown by step but could be detrimental to CO2 clearance (a very serious concern in COPD patients).

Thus, for optimal results, both SpO2 and CO2 concentrations (or Ventilation) may be monitored for optimization. Ventilation is shown as monitored instep 78, in particular using capnography based on different features and phases of the capnograph. SpO2 is shown as monitored in step 79.

In this way, monitoring both CO2 and SpO2 can be used to help determine optimal PEEP. In its present form, controlling only Fi02 and monitoring only SpO2 does not guarantee optimal PEEP level supports. Pressure support is viable for HFNT but optimizing this value could be greatly enhanced via having both gases.

Increasing PEEP would increase SpO2, however it can also increase dead space and ventilation / perfusion mismatch. This can be detected with reduced CO2 clearance. Thus, by monitoring both CO2 and SpO2 an optimal PEEP level can be achieved.

It is recently known that capnography can measure intrinsic PEEP, iPEEP. iPEEP measurements are critical especially in COPD patients. iPEEP increases hyperinflation and patient efforts. iPEEP could be eliminated by adjusting to the right PEEP pressure. Thus, optimal PEEP could be further achieved by considering iPEEP values. In other words, when such knowledge is available, then the decision of these two flows could enhance personalized therapy across different patient conditions (i.e. hypoxemia and hypercapnia) and the management of resources especially of oxygen at home settings.

Fig. 7 shows how controlled parameters (oxygen flow $F_{O2}(t)$, air flow $F_D(t)$ and total flow $F_T(t)$) may be adjusted according to changes in targeted parameters.

The top plot shows the oxygen saturation SpO2, the second plot shows the partial pressure PaCO2 of arterial CO2, the third plot shows the nasal pressure, the fourth plot shows the respiratory rate RR and the bottom plot shows the HFNT flows (oxygen, air and total). All plots are over time.

In addition to PEEP, target ranges 80, 82, 84, 86 for SaO2, PaCO2, Nasal pressure, and respiratory rate (RR) could be set. Then, the air flow and Fi02 are varied to obtain the set target ranges 80, 82, 84, 86. When any targeted parameter falls outside the set range, the correct control is followed on either the device and/or oxygen flow to return the therapy to the required target.

In measuring the parameters of interest ("targeted parameters"), PaCO2 is estimated from Capnography or Transcutaneous CO2 measurement, SaO2 from pulse oximetry, nasal pressure from a pressure sensor at nasal cannula and RR from any of these methods: swings in nasal cannula measurements, pulse oximetry, or capnography.

Thus a device or method according to the invention may employ one or more of the following, in any combination: estimation of PaCO2 from capnography or transcutaneous CO2 measurements, SaO2 measurement from pulse oximetry, nasal pressure measurement from a pressure sensor or nasal cannula and RR from any of these methods. Alternatives to the specified methods or sensors may be employed, in any combination.

The air flow $F_D(t)$ and oxygen flow $F_{O2}(t)$ (based on Fi02) may be controlled simultaneously to achieve the target range under various different scenarios:

With a drop of SaO2 oxygen flow $F_{O2}(t)$ is increased while air flow $F_D(t)$ is decreased maintaining same total flow (vertical dashed line 90);

With an increase of PaCO2, air flow and oxygen flow $F_{O2}(t)$ are increased to maintain same Fi02 but help in CO2 clearance (vertical dashed line 92); and

With a drop in nasal pressure below a threshold, the total bulk flow is increased to compensate for the loss in pressure and so is oxygen flow to keep Fi02 constant (vertical dashed line 94).

Similarly, with an increase in RR, air flow and oxygen flow $F_{O2}(t)$ are increased. (vertical dashed line 96).

It is possible that several stable states could be achieved through this process. Stable states are defined when the parameters fall within the accepted ranges defined by thresholds that are either based on group population in relationship to the patient condition, or set by clinicians particularly for the patient. Moreover, the user could help set these thresholds by noting at which states (or parameter settings) he or she is most comfortable. With these selections the algorithm could better approach these comfortable settings.

Fig. 7 thus provides an illustrative example of how measuring these different variables allows therapy to be optimized. While PaCO2 is shown in this example, other features of the Capnograph such as dead space volume could be utilized as well for optimization.

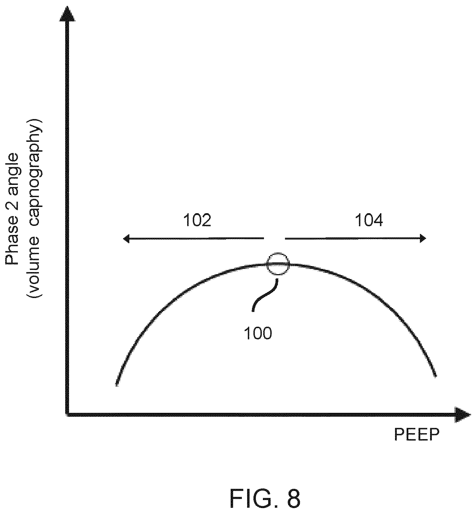

Fig. 8 shows a volume capnography plot of phase 2 angle versus PEEP, which illustrates one example where the angle of phase 2 from the volume capnograph could help in optimizing PEEP. As PEEP decreases, for example from an optimal point 100 in the direction shown by arrow 102, there is not enough pressure support. As PEEP increases from the optimal point 100 in the direction shown by arrow 104, there is increasing dead space. The higher the angle of phase II, the better the ventilation perfusion ratio and the less volume is lost in dead space.

At the optimal point 100, the PEEP provides appropriate pressure support for the patient to help in clearing out CO2 but without increasing the dead space significantly. This provides an example of using a Capnograph feature other than a PaCO2 estimate to help optimize therapy. Optimal PEEP 100 could be obtained by optimizing between providing the right pressure support and avoiding increased dead space.

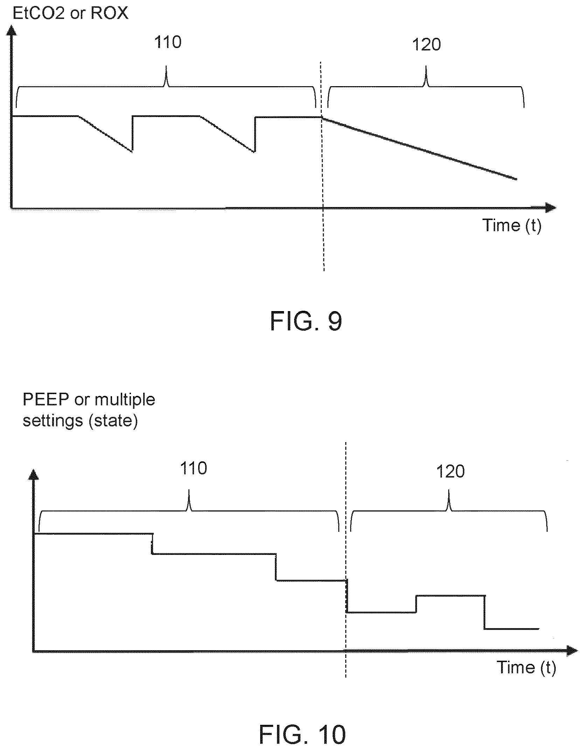

Fig. 9 shows a plot of EtCO2 or ROX over time.

Fig. 10 shows a plot of PEEP or multiple settings over time.

Fig. 9 and Fig 10 show how the invention could be applied to longer term adaption of treatment. As shown in both Fig. 9 and Fig. 10, a period of efficient treatment is shown 110, followed by a period 120 during which the treatment is inefficient and therapy needs to be transitioned. As shown in Fig. 9, a single parameter (e.g. EtCO2) or an index (ROX) could be monitored across time. As shown in Fig 10, parameters (e.g. PEEP) or a set of parameters or multiple settings (defined as a state) could be tracked and changed (the changes are not shown) to return treatment to the required threshold, following a deterioration in the monitored value (s). It has been demonstrated that after the first two deteriorations the HFNT therapy can be adapted to continue efficient treatment. However, following that, despite several attempts from the device to achieve the required air flow, oxygen flow and total flow settings there was continuous deterioration. Thus, this could indicate that the therapy is no longer adequate for the patient, and an intervention is needed.

Two indexes, which are a combination of parameters, could be calculated and used to monitor the progression of the patient condition on a long-time scale as well as support in parameter estimation on a short-time scale.

For example, the two indexes may be the rapid shallow breathing index (RSBI) and the ROX index. These two indexes are equated as follows:

$$RSBI = RR/VT$$

$$ROX = \frac{\frac{SpO2}{FiO2}}{RR}$$

Where VT is the tidal volume. While RSBI is mainly intended for weaning prediction (success or failure), in its basic form it captures the ability of the patient to be able to support normal breathing demands (physiological tidal volumes from physiological patient efforts/breathing rate). The lower the value the better the patient condition is in general. Higher ROX values on the other hand indicate better condition as they demonstrate proper oxygenation at lower patient effort in general. It is possible to combine these two in one new index, the ROX/RSBI index:

$$\frac{ROX}{RSBI} = \frac{VT\frac{SpO2}{FiO2}}{RR^2}$$

At the beginning of the treatment, the baseline value for either or both could be measured and monitored on a daily or weekly basis to determine if the patient is benefiting from the therapy or not. In case not, this indication could be very helpful to suggest the need to step up therapy (e.g. going from oxygen support to a CPAP or ventilator).

On the shorter-term as mentioned above this could help in deciding on the optimal flow and Fi02. For example, if there is a drop of this combined index in parallel with a drop in SpO2, then Fi02 could be increased to attempt to increase SpO2 in such a way the overall combined index increase. If that succeeds, then the new settings are fine. If it fails, then the total flow could be increased to provide more pressure support and reduce patient effort to ultimately increase the combined index.

The features, parameters, and indexes presented above could be also used to track the progression of a patient in relationship to therapy and help determine when potentially a change in therapy is required (e.g. COPD patient transitioning from HFNT to non-invasive ventilation, NIV), and could be employed in a similar manner to the example provided in Fig. 7. The insight provided is also used for therapy optimization; however, in this case, it is also used to look at the longer time frame compared to direct control of HFNT settings. Additionally, from this monitoring, which also includes capturing the change in the capnography waveform, the disease progression (e.g. transition between COPD stages) can be captured. This will help step up the therapy and/or vary the threshold parameters set by the therapist.

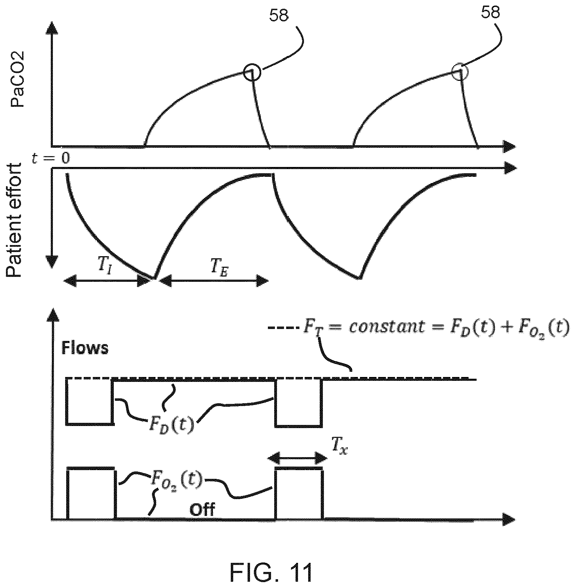

Fig. 11 shows as the top plot a capnograph of PrCO2, as the middle plot patient effort and as the bottom plot oxygen flow $F_{O2}(t)$ and air flow $F_D(t)$ over time, and shows how the capnograph may be used to detect inhalation and exhalation in order to optimize HFNT.

$T_i$ is the inspiration time; $T_E$ is expiration time and $T_x$ is the time when oxygen flow is stopped. $0 < T_x < T_i$.

The total flow $F_T$ is here maintained as a constant, and is the summation of air flow $F_D(t)$ and oxygen flow $F_{O2}(t)$. Detecting inhalation-exhalation (IE) state in HFNT is critical as it allows the control of the air and oxygen flows to synchronize with the patient's breathing (e.g. deliver oxygen only during inspiration, deliver different flow values in inspiration and expiration to achieve different pressure support levels such as a BiPAP (bilevel positive airway pressure). By determining when PCO2 levels start to rise and determining when EtCO2 is reached (point 58), the capnograph may be used to detect IE state and thus aid in the control of oxygen flow $F_{O2}(t)$ and total flow $F_T(t)$ (e.g. providing oxygen flow only during inspiration as shown). Although the figure exemplifies a constant flow, that is not necessarily the case, the flow could be adjusted to achieve a certain pressure (e.g. PEEP, IPAP, EPAP), SpO2, EtCO2, or a combination of these. Also plotted against the same time period is the expected patient effort over time, according to the stage of inhalation or exhalation.

During patient inhalation the capnograph signal is zero, while at the onset of exhalation the signal increases. This does not only allow the detection of the IE state but also allows the timing of oxygen delivery and the control of the total flow to be precisely controlled. This may allow oxygen to be conserved, and thus save cost and reduce the servicing intervals required to change oxygen. As observed in the figure, oxygen is delivered for a time $T_x$ ($T_x < T_I$). During that time, the air flow is reduced, and later when oxygen delivery is stopped, the air flow compensates to maintain the same flow rate. To set $T_x$, the exact inspiration time $T_I$, requires to be known at the beginning of the inspiration period or the patient's effort. This is not possible; thus, it could be continuously estimated from several previous cycles (e.g. from the previous 50 breaths). When the system starts up, it could first set $T_x = T_I$.

The capnograph signal could also help in indicating the comfort of the patient or if the flow settings are not adequate or synchronized to the patient breathing cycle. To give an example, during exhalation, the total net flow should be flowing from the patient and the device should support the patient exhalation. This could be done by reducing PEEP to help the patient in exhaling. Thus, detection of those cases could help therapy optimization. In the example presented, and as an example solution, the overall flow could be reduced during exhalation to avoid this dip.

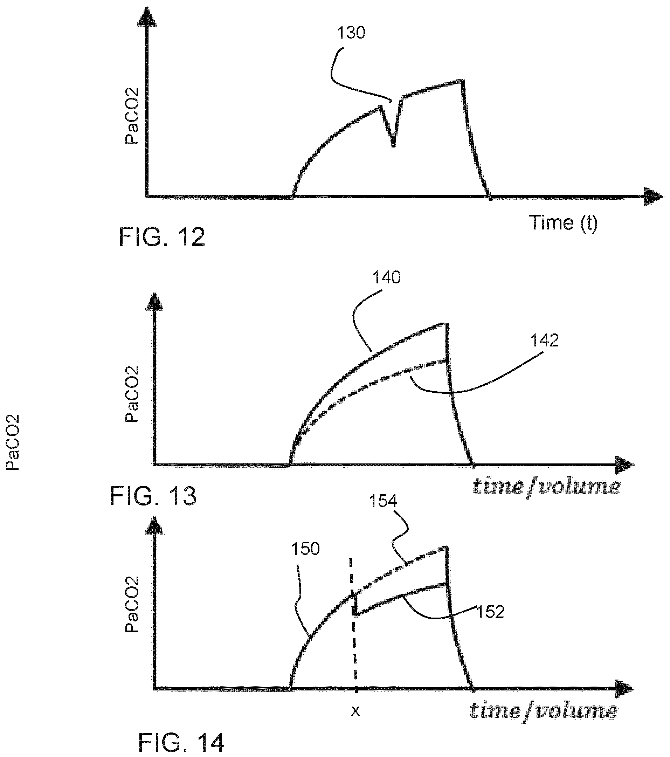

Fig. 12 shows a capnograph with an indent or dip 130, which may be indicative of patient discomfort. This patient discomfort may be due to improper HFNT settings (e.g. flow setting) or incorrect synchronization of the delivered flow with the patient breathing cycle (e.g. increased flow during exhalation instead of decreasing flow) in order to reduce PEEP or achieve an EPAP lower than IPAP to support exhalation. Monitoring the features and shape of the capnograph may therefore assist in correcting the HFNT settings or synchronization of the air flow with the patient breathing cycle. This correction may be automatic by means of the microcontroller 26 directly adjusting settings through predetermined algorithms, or a signal may be used to alert a therapist who may adjust settings manually.

Fig. 13 shows a capnograph with the PrCO2 plot of two breaths superposed, which may be useful to estimate patient flow utilizing dilution. Consecutive breath cycles 140, 142 could be used to estimate dilution. While dilution is generally regarded as a negative effect on the therapy, it might be possible to utilize dilution to estimate the patient flow and help in assessing the patient condition or optimal device settings. The estimate could be obtained using a minimum of two capnograph waveforms 140, 142 each carried out using a different total flow setting and preferably consecutively.

The assumptions are that the patient effort and flow would be similar across these two or more consecutive breaths as well as the exhaled CO2. Rewriting this numerically for the first flow setting (1) and the second flow setting (2):

$$CO2_{capno,1}(t) = \frac{F_P(t) * CO2_P(t)}{F_{T,1} + F_P(t)}$$

$$CO2_{capno,2}(t) = \frac{F_P(t) * CO2_P(t)}{F_{T,2} + F_P(t)}$$

$F_P$ is the patient flow and $F_T$ is the therapy total flow.

$CO2_{capno}$ is the CO2 concentration read from a capnograph and $CO2_P$ is the exhaled patient CO2 concentration (not effected by dilution).

By dividing these two ratios

$$\frac{CO2_{capno,2}}{CO2_{capno,1}} = \frac{F_P(t) * CO2_P(t)}{F_{HFNT,2} + F_P(t)} \frac{F_{HFNT,1} + F_P(t)}{F_P(t) * CO2_P(t)} = \frac{F_{HFNT,1} + F_P(t)}{F_{HFNT,2} + F_P(t)} = \beta$$

Where $\beta$ is the ratio between the two flows and may be obtained graphically. Finally, patient flow could be estimated as:

$$F_P = \frac{\beta F_{T,2} - F_{T,1}}{1 - \beta}$$

Potentially if the device was off during one of those measurements (i.e. $F_{T,1} = 0$) the equation is further simplified:

$$F_P = \frac{\beta F_{T,2}}{1 - \beta} \qquad \text{for } F_{T,1} = 0$$

The change in the total flow should be sufficient to produce a significant $\beta$ in order to estimate the patient flow. However, at the same time the change should not be too large to significantly affect the patient flow (assumed to be the same). Thus, a lower and upper threshold exist for $\beta$:

$$\beta_{Threshold,lower} \leq \beta \leq \beta_{Threshold,upper}$$

Note that $\beta \leq 1$ if $F_{T,2} > F_{T,1}$ or $\beta \geq 1$ if $F_{T,2} < F_{T,1}$. In other words, the method works either going from a lower to a higher flow or vice versa.

Fig. 14 shows a capnograph with an extrapolated PrCO2 plot, depicting an alternative method of determining dilution. Thus, rather than using two different cycles, one cycle could be utilized. In this case, the flow is increased during the middle of the exhalation phase (at time/volume = x), leading to dilution from that point and thus a lower PrCO2 flow. Then the shape of the initial curve 150 is extrapolated to give curve 154. This predicted portion 154 is then used with the measured flow 152 (after time/volume = x) to estimate patient flow by determining the difference between extrapolated flow 154 and measured flow 152.

An additional embodiment comprises a method that would allow the calibration of the capnograph waveform by utilizing the oxygen flow. Since the device controls the oxygen concentration or Fi02 (e.g. 70%, 80%...), by simply adding a sensor to measure the oxygen concentration near the capnograph the calibration factor could be obtained. In other words, both the O2 and the CO2 should be diluted similarly because they are mixing in the same net flow. Thus, by knowing the dilution factor of one, the other could be known. This method could be the simplest as it only requires an additional sensor to measure the oxygen concentration at the mixed air. However, this assumes that no oxygen is exhaled by the patient (or all oxygen intake is consumed) or comes from any other additional source (e.g., entrapment from the atmosphere).

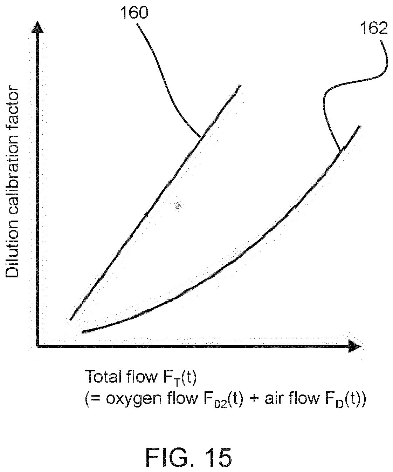

Fig. 15 is a plot showing example relationships between a dilution calibration factor (y-axis) and total flow (x-axis). The relationship could be linear or non-linear as exemplified by the two plots (calibration curves 160, 162), but in general the dilution calibration factor will increase as flow increases as more flushing (dilution of flow) occurs at higher flows.

Where capnography is employed with HFNT, there may be a dilution from the flow delivered by the HFNT device, affecting the capnography measurements. It may be desirable to compensate for this effect.

A method of compensating for this dilution is to compare the capnograph waveform during HFNT operation and without. For instance, the HFNT device could be on and one or more capnograph waveforms be collected. Then the device could be switched off during exhalation only and one or more further capnograph waveforms be collected. A dilution factor, which may also be called a calibration factor, could then be obtained, by comparing the waveform before and after pausing the device and determining the ratio between the two.

This process of generating a calibration factor at a given flow may be repeated at different flows to generate a calibration curve, which is a function of dilution calibration factor against total flow. The calibration curve could then be utilized during operation. This process of generating a calibration curve may be repeated after changes in the total flow, as well as every few days or weeks in case the patient effort profile has changed. The calibration curve could be obtained at device setup and the appropriate dilution/compensation factor could be selected from the curve, based on the flow setting. It may be desirable to obtain a calibration curve at the beginning of the therapy (setting up the device), and this could be repeated every few months to update the calibration in case it has changed.

Alternatively, the dilution during exhalation could be accounted for by utilizing basic knowledge in gas concentrations following flow mixing. To elaborate, a flow sensor could measure the mixed flow ($F_{mixed}$), which is the summation of the total flow ($F_T$) and the patient flow (Fp). $F_{mixed}$ can be written as:

$$F_{mixed} = F_P + F_T$$

The flow that passes through or is sampled by the capnograph should be the mixed flow, $F_{mixed}$. Thus, the concentration read by the capnograph is the weighted average of the concentration from each flow ($F_T$ and $F_P$) divided by $F_{mixed}$. Since $F_T$ has no CO2, the concentration read by the capnograph ($CO2_{capno}$) in relation to the flows and exhaled patient CO2 concentration ($CO2_P$) during exhalation is:

$$CO2_{capno}(t) = \frac{F_P(t) * CO2_P(t)}{F_{mixed}(t)} = \frac{(F_{mixed} - F_T) * CO2_p}{F_{mixed}}$$

Rearranging, the actual patient exhaled CO2 concentration is:

$$CO2_P(t) = \frac{F_{mixed}(t) * CO2_{capno}(t)}{F_{mixed}(t) - F_T(t)} = \frac{1}{1 - \frac{F_T(t)}{F_{mixed}(t)}} CO2_{capno}(t) = K * CO2_{capno}(t)$$

Where the calibration factor is K:

$$K(t) = \frac{1}{1 - \frac{F_T(t)}{F_{mixed(t)}}} = \frac{\frac{F_{mixed}(t)}{F_{T(t)}}}{\frac{F_{mixed}(t)}{F_{T(t)}} - 1}$$

Note that the generated calibration factor K(t) is a function of time and can directly adjust to changes in air flow as well as net flow. This is valuable since the reason flow could change during the exhalation phase (or across time) could be to achieve a particular pressure support value (e.g. EPAP). K(t) is also always larger than one since the capnograph underestimates exhaled CO2.

In general, the dilution could affect the values of the capnograph but not its shape. In other words, it dilutes the EtCO2 value and thus the estimate of PaCO2. Thus, one can still utilize the shape of the capnograph (e.g. slopes, plateau, and the other features discussed) to optimize therapy and track the disease without the need to factor in for dilution.

In addition to the control of flow as described above, the controller also may be adapted to control a humidifier to adjust the humidity of the breathing gas according to the measurement or estimation of a CO2 level, and/or the controller may be adapted to control a heater to adjust the temperature of the breathing gas. Accordingly, the air is delivered at the appropriate temperature and humidity to the patient mainly for comfort.

There are also further possible functions of the therapy system. The system could potentially pause the delivery of flow (both from the device and the oxygen) and take several breath waveforms without the effect of the device in any way. These breaths could be used to monitor the progression of the disease without any device effect as well as analyze the effect of the HFNT settings on the patient parameters (e.g. impact of total flow on respiratory rate of patient, or how the slopes of the capnograph are affected by HFNT.) The insight is fundamental to understand how HFNT is behaving and how it could be controlled to achieve optimal settings.

Additionally, if the patient has his/her mouth closed and a flow sensor is detecting the full exhalation flow from the nose, then the volume capnography can be achieved. As explained earlier, this provides more detailed information than time capnography capturing for example dead space volume. If additionally, a flow sensor is used in conjunction with a mouthpiece having a controllable pinhole, then continuously constructing the volume capnography is still possible given flow sensors and capnographs at both exhalation sites (nose and mouth).

Additionally, if pressure is measured at these additional sites then respiratory resistance (R) and respiratory compliance (C) could be estimated with the help of an algorithm Monitoring R and C are key in detecting the progression of the disease and can also help in optimizing the flow settings. Both R and C are functions of these settings and finding the optimal values would ensure a better therapy (e.g. potentially ventilation-induced lung injury could be avoided at very high flows)

Finally, the device could also be used to detect when the nasal cannula is not properly detected or totally disconnected. In case the exhaled CO2 (from the capnograph) is zero for an elongated time, then a warning could be provided to the user to properly set the nasal cannula or put it on. In case of no response or change in few minutes, then most probably the patient is not using the device and it could be turned off automatically.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

**Claims**

1. A therapy system, comprising:

   a patient interface (12) adapted to deliver gas to a nasal cavity of a patient;
   a delivery system (16) for delivering an oxygen-enriched breathing gas to the patient interface, wherein the delivery system is adapted to deliver a total flow rate of breathing gas comprising an oxygen flow rate and an air flow rate, wherein the delivery system is controllable to adjust one or more of the total flow rate, the oxygen flow rate and the air flow rate;
   a controller (26) for controlling the delivery system;
   a sensor arrangement (30) adapted to enable measurement or estimation of the arterial partial pressure of arterial carbon dioxide, PaCO2, of the patient over time and thereby generate capnograph data; and

wherein the controller (26) is adapted to:
iteratively:

determine a parameter from the capnograph data, which parameter is representative of $CO_2$ washout;
adjust one or more of the total flow rate, the oxygen flow rate or the air flow rate of the breathing gas delivered by the delivery system; and
determine the impact of the adjusting upon the determined parameter, in order to determine a value of total flow rate, oxygen flow rate or air flow rate which maximizes $CO_2$ washout or achieves a desired level of $CO_2$ washout; and

set the total flow rate, oxygen flow rate or air flow rate at the determined value.

2. The system of claim 1 wherein:

the measured parameter from the capnograph data is the end-tidal $CO_2$ concentration, $EtCO_2$; and
the controller is adapted to determine the value of total flow rate, oxygen flow rate or air flow rate for maximizing $CO_2$ washout by maximizing the value of $EtCO_2$.

3. The system of any preceding claim wherein:

the measured parameter of the capnograph data is the phase two angle; and
the controller is adapted to determine the value of total flow rate, oxygen flow rate or air flow rate to optimize a positive end-expiratory pressure, PEEP, thereby to provide the maximum phase two angle, in order to achieve a maximum $CO_2$ washout.

4. The system of any preceding claim wherein the controller is further adapted to:

derive, from the measurement or estimation of the arterial partial pressure, a patient parameter or index;
monitor the patient parameter or index over time to detect or monitor a patient condition; and
adjust one or more of the total flow rate, the oxygen flow rate or the air flow rate of the breathing gas delivered by the delivery system in dependence on the detected or monitored patient condition.

5. The system of claim 4 wherein the monitoring the patient parameter or index over time comprises monitoring one or more of an end-tidal $CO_2$ concentration, $EtCO_2$, a rapid shallow breathing index, RSBI, or a ratio of $SpO_2$ / $Fi0_2$ to respiratory rate, ROX index.

6. The system of any preceding claim wherein the sensor arrangement is further adapted to measure or estimate $SpO_2$ over time, and the controller is further adapted to:

receive or determine a target $SpO_2$ level; and
adjust one or more of the total flow rate, oxygen flow rate or air flow rate of the breathing gas in dependence on measured or estimated $SpO_2$ level to achieve the target $SpO_2$ level.

7. The system of claim 6 wherein the controller is adapted to:

first adjust the air flow to achieve the optimal air flow rate for maximum $CO_2$ washout; and
then adjust the oxygen flow to achieve the target $SpO_2$ level.

8. The system of any preceding claim wherein the controller is adapted to compensate for dilution of the flow by constructing adjusted capnograph data and estimating a calibration factor, wherein:

the estimating a calibration factor comprises generating a first set of capnograph data and a second set of capnograph data;
the controller is adapted to control the sensor arrangement and the total flow rate of the delivery system to generate the first set of capnograph data and the second set of capnograph data at different flows; and
the calibration factor is determined by comparing the first set of capnograph data and the second set of capnograph data.

9. The system of claim 8 wherein the controller is adapted to:
control the sensor arrangement and the total flow rate of the delivery system to generate either the first set of capnograph data or the second set of capnograph data when the total flow is zero.

10. The system of any preceding claim wherein the sensor arrangement is adapted to measure an intrinsic PEEP, iPEEP, level and wherein the controller is adapted to determine a target positive end-expiratory pressure, PEEP, taking into account the measured iPEEP level.

11. A computer-implemented method for controlling the delivery of gas to a nasal cavity of a patient via a patient interface, the method comprising:

controlling a delivery system to deliver an oxygen-enriched breathing gas to the patient interface, thereby delivering a total flow rate of breathing gas comprising an oxygen flow rate and an air flow rate;
iteratively:

receiving capnograph data from a sensor arrangement;
determining a parameter from the capnograph data, which parameter is representative of CO2 washout;
adjusting one or more of the total flow rate, the oxygen flow rate or the air flow rate of the breathing gas delivered by the delivery system; and
determining the impact of the adjusting upon the determined parameter, in order to determine a value of total flow rate, oxygen flow rate or air flow rate which maximizes CO2 washout or achieves a desired level of CO2 washout; and

setting the total flow rate, oxygen flow rate or air flow rate at the determined value.

12. A computer-implemented method for controlling the delivery of gas to a nasal cavity of a patient via a patient interface according to claim 11,

wherein the determined parameter from the capnograph is the end-tidal CO2 concentration, EtCO2, and the method comprises
determining the optimal total flow rate, oxygen flow rate or air flow rate for maximizing CO2 washout by maximizing the value of EtCO2.

13. A computer-implemented method for controlling the delivery of gas to a nasal cavity of a patient via a patient interface according to claim 11 or claim 12,

wherein the determined parameter from the capnograph is the phase two angle, and the method comprises determining the optimal total flow rate, oxygen flow rate or air flow rate for maximizing CO2 washout by optimizing the PEEP by maximizing the phase two angle.

14. A computer program comprising computer program code means which is adapted, when said program is run on a computer, to implement the method of any of claims 11 to 13.

FIG. 1
PRIOR ART

FIG. 2

FIG. 3

FIG. 4

FIG. 5

FIG. 6

FIG. 7

FIG. 8

FIG. 9

FIG. 10

FIG. 11

FIG. 12

FIG. 13

FIG. 14

160

162

Dilution calibration factor

Total flow $F_T(t)$
(= oxygen flow $F_{02}(t)$ + air flow $F_D(t)$)

FIG. 15

Europäisches
Patentamt

European
Patent Office

Office européen
des brevets

# EUROPEAN SEARCH REPORT

Application Number

EP 22 17 1395

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | AU 2021 203 928 A1 (FISHER & PAYKEL HEALTHCARE LTD [NZ]) 8 July 2021 (2021-07-08) | 1,2,4,5, 11,12,14 | INV. A61M16/00 A61M16/06 |
| Y | * figures 1, 2 * * paragraphs [0023], [0035], [0036] * | 3,8-10, 13 | A61M16/12 A61M16/10 |
| X | US 2018/126110 A1 (PAYTON MATTHEW JON [NZ] ET AL) 10 May 2018 (2018-05-10) * figure 1 * * paragraphs [0148] – [0184], [0214] – [0229], [0256] – [0260] * | 1,2,4-7, 11,12,14 | |
| X | US 2018/104426 A1 (OLDFIELD SAMANTHA DALE [NZ] ET AL) 19 April 2018 (2018-04-19) * figure 1 * * paragraphs [0114], [0130] – [0142], [0308] – [0319] * | 1,2,4-7, 11,12,14 | |
| X | CN 111 991 663 A (JIANGXI ANLIAN MEDICAL EQUIPMENT CO LTD) 27 November 2020 (2020-11-27) * paragraphs [0005] – [0010], [0014], [0015] * | 1,2,4,5, 11,12,14 | TECHNICAL FIELDS SEARCHED (IPC) A61M |
| Y | WO 2021/101780 A1 (INCOBA LLC [US]) 27 May 2021 (2021-05-27) * figure 1 * * paragraphs [0035], [0036] * | 3,13 | |
| Y | US 2020/383606 A1 (EVANS ALICIA JERRAM HUNTER [NZ] ET AL) 10 December 2020 (2020-12-10) * paragraphs [0096] – [0103] * | 8,9 | |
| Y | US 2022/105288 A1 (BECK GEORGE [US] ET AL) 7 April 2022 (2022-04-07) * paragraphs [0363], [0364] * | 10 | |

The present search report has been drawn up for all claims

| Place of search | Date of completion of the search | Examiner |
|---|---|---|
| Munich | 24 October 2022 | Trattner, Barbara |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons
...........................................................................
& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 22 17 1395

This annex lists the patent family members relating to the patent documents cited in the above-mentioned European search report.
The members are as contained in the European Patent Office EDP file on
The European Patent Office is in no way liable for these particulars which are merely given for the purpose of information.

24-10-2022

| Patent document cited in search report | | Publication date | Patent family member(s) | | Publication date |
|---|---|---|---|---|---|
| AU 2021203928 | A1 | 08-07-2021 | AU 2015340118 | A1 | 18-05-2017 |
| | | | AU 2021203928 | A1 | 08-07-2021 |
| | | | EP 3212263 | A1 | 06-09-2017 |
| | | | US 2018008791 | A1 | 11-01-2018 |
| | | | US 2020390992 | A1 | 17-12-2020 |
| | | | WO 2016068725 | A1 | 06-05-2016 |
| US 2018126110 | A1 | 10-05-2018 | EP 3242703 | A1 | 15-11-2017 |
| | | | US 2018126110 | A1 | 10-05-2018 |
| | | | WO 2016133406 | A1 | 25-08-2016 |
| US 2018104426 | A1 | 19-04-2018 | AU 2016241573 | A1 | 12-10-2017 |
| | | | AU 2021202228 | A1 | 06-05-2021 |
| | | | CA 2980849 | A1 | 06-10-2016 |
| | | | CN 107614042 | A | 19-01-2018 |
| | | | CN 114344648 | A | 15-04-2022 |
| | | | CN 114392441 | A | 26-04-2022 |
| | | | EP 3259001 | A1 | 27-12-2017 |
| | | | GB 2552626 | A | 31-01-2018 |
| | | | JP 7002940 | B2 | 20-01-2022 |
| | | | JP 2018512949 | A | 24-05-2018 |
| | | | JP 2022050509 | A | 30-03-2022 |
| | | | KR 20180008435 | A | 24-01-2018 |
| | | | US 2018104426 | A1 | 19-04-2018 |
| | | | WO 2016157106 | A1 | 06-10-2016 |
| CN 111991663 | A | 27-11-2020 | NONE | | |
| WO 2021101780 | A1 | 27-05-2021 | US 2022265164 | A1 | 25-08-2022 |
| | | | WO 2021101780 | A1 | 27-05-2021 |
| US 2020383606 | A1 | 10-12-2020 | EP 3177207 | A1 | 14-06-2017 |
| | | | US 2017281051 | A1 | 05-10-2017 |
| | | | US 2020383606 | A1 | 10-12-2020 |
| | | | WO 2016035035 | A1 | 10-03-2016 |
| US 2022105288 | A1 | 07-04-2022 | US 2022105288 | A1 | 07-04-2022 |
| | | | WO 2022076407 | A2 | 14-04-2022 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20110257550 A

**Non-patent literature cited in the description**

- **COLMAN, YEHOSHUA ; BARUCH KRAUSS.** *Microstream Capnography Technology: A New Approach to an Old Problem,* vol. 7